(19) Europäisches Patentamt / European Patent Office / Office européen des brevets

(11) **EP 3 375 886 A1**

(12) **EUROPEAN PATENT APPLICATION**
published in accordance with Art. 153(4) EPC

(43) Date of publication:
**19.09.2018 Bulletin 2018/38**

(51) Int Cl.:
*C12Q 1/68* (2018.01)   *C12N 15/09* (2006.01)
*C12Q 1/6876* (2018.01)   *G06F 19/14* (2011.01)

(21) Application number: **16863998.7**

(22) Date of filing: **24.10.2016**

(86) International application number:
**PCT/JP2016/081476**

(87) International publication number:
**WO 2017/082034 (18.05.2017 Gazette 2017/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **10.11.2015 JP 2015220598**

(71) Applicant: **Fujifilm Corporation
Minato-ku
Tokyo 106-8620 (JP)**

(72) Inventor: **TSUJIMOTO Takayuki
Kanagawa 258-8538 (JP)**

(74) Representative: **Klunker IP
Patentanwälte PartG mbB
Destouchesstraße 68
80796 München (DE)**

(54) **METHOD FOR DETERMINING WHETHER CELLS OR CELL MASSES ARE ORIGINATED FROM SAME PERSON, OR UNRELATED PERSONS, OR PARENT AND CHILD, OR RELATED PERSONS**

(57) Provided is a method for determining whether cells or cell groups that have a possibility of being derived from a plurality of persons are derived from the same person, or unrelated persons, or a parent and a child, or persons in a blood relationship, the method including: a step of acquiring genotypic data for gene polymorphism sites of the cells or cell groups and weighting the data with a preset weight distribution, to acquire weighted genotypic data; and a step of using the weighted genotypic data to determine whether the cells or cell groups are derived from the same person, or unrelated persons, or a parent and a child, or persons in a blood relationship, in which the preset weight distribution is set by mapping of a measured apparent genotype and a true genotype with respect to the gene polymorphism sites, and the method being capable of determining, with high accuracy, whether the cells or cell groups are derived from the same person, or unrelated persons, or a parent and a child, or persons in a blood relationship, even in a case where errors due to DNA amplification and sequencing are large and errors frequently occur in thus obtained polymorphism information of genes in genetic analysis for a small amount of deoxyribonucleic acid (DNA).

**EP 3 375 886 A1**

**Description**

**BACKGROUND OF THE INVENTION**

1. Field of the Invention

The present invention relates to a method for determining whether cells or cell groups are derived from the same person, or unrelated persons, or a parent and a child, or persons in a blood relationship.

2. Description of the Related Art

It is known that there are fetal cells, which have been introduced into maternal veins from a placenta, in peripheral blood of a pregnant woman. By examining chromosomes or genes of such fetal cells, non-invasive prenatal diagnosis of fetal chromosomal abnormalities or genetic abnormalities can be performed.

An existence frequency of fetal cells in peripheral blood of a pregnant woman is about one in $10^5$ to $10^7$ as a frequency of nucleated spheres, and it is also difficult to distinguish fetal cells from maternal cells from their appearance. Thus, there is a high possibility that maternally derived cells are incorporated in fetally derived cells or cell groups which are obtained from maternal blood by extraction. Therefore, in prenatal diagnosis based on fetal cells in peripheral blood of a pregnant woman, it is necessary to discriminate whether cells to be examined are derived from a fetus or a mother, based on genetic information.

However, the amount of deoxyribonucleic acid (DNA) contained in a single cell is small. Currently, sequencing technology and bioinformatics technology for such a small amount of DNA are in the process of development. Since errors due to DNA amplification and sequencing are large, errors frequently occur in thus obtained polymorphism information of genes. For example, due to errors during DNA amplification or sequencing, allelic drop-out, allelic drop-in, and the like may occur, and alleles that should exist may not be detected, or alleles that should not exist may be detected. As a result, it is difficult to accurately determine whether each cell is derived from a fetus or a mother.

On the other hand, WO2012/088456A discloses a method for non-invasive prenatal paternity testing which uses genetic measurements acquired based on plasma taken from a pregnant mother, along with genetic measurements of an alleged father, and genetic measurements of a mother, to determine whether or not the alleged father is a biological father of a fetus. This method is a method of collecting peripheral blood of the alleged father and the pregnant mother and using genetic information such as a single nucleotide polymorphism (SNP) contained in the blood to perform an evaluation. That is, such method is a method of estimating a chance that the alleged father is a biological father, from DNA of the alleged father and mixed DNA of the mother and the fetus.

**SUMMARY OF THE INVENTION**

However, in prenatal diagnosis based on fetal cells in peripheral blood of a pregnant woman, it is necessary to distinguish between fetal cells and mother cells on a cell-by-cell basis. Thus, the technique disclosed in WO2012/088456A cannot be applied as it is.

Accordingly, an object of the present invention is to provide a method for determining whether cells or cell groups that have a possibility of being derived from a plurality of persons are derived from the same person, or unrelated persons, or a parent and a child, or persons in a blood relationship, the method being capable of determining, with high accuracy, whether the cells or cell groups are derived from the same person, or unrelated persons, or a parent and a child, or persons in a blood relationship, even in a case where errors due to DNA amplification and sequencing are large and errors frequently occur in thus obtained polymorphism information of genes in genetic analysis for a small amount of deoxyribonucleic acid (DNA).

As a result of intensive investigations to achieve the object, the present inventors have found that by performing a method for determining whether cells or cell groups are derived from the same person, or unrelated persons, or a parent and a child, or persons in a blood relationship, including a step of acquiring genotypic data for gene polymorphism sites of the cells or cell groups and weighting the data with a preset weight distribution, to acquire weighted genotypic data; and a step of using the weighted genotypic data to determine whether the cells or cell groups are derived from the same person, or unrelated persons, or a parent and a child, or persons in a blood relationship, in which the preset weight distribution is set by mapping of a measured apparent genotype and a true genotype with respect to the gene polymorphism sites, it is possible to determine, with high accuracy, whether the cells or cell groups are derived from the same person, or unrelated persons, or a parent and a child, or persons in a blood relationship, even in a case where errors due to DNA amplification and sequencing are large and errors frequently occur in thus obtained polymorphism information of genes in genetic analysis for a small amount of deoxyribonucleic acid (DNA), and thus have completed the present invention.

That is, the present invention is set forth in following [1] to [9].

[1] A method for determining whether cells or cell groups that have a possibility of being derived from a plurality of persons are derived from the same person, or unrelated persons, or a parent and a child, or persons in a blood relationship, the method comprising:

a step of acquiring genotypic data for gene polymorphism sites of the cells or cell groups and weighting the data with a preset weight distribution, to acquire weighted genotypic data; and

a step of using the weighted genotypic data to determine whether the cells or cell groups are derived from the same person, or unrelated persons, or a parent and a child, or persons in a blood relationship, in which the preset weight distribution is set by mapping of a measured apparent genotype and a true genotype with respect to the gene polymorphism sites.

[2] The method described in [1], in which the mapping of the apparent genotype and the true genotype is estimated by experiments and/or simulations using a plurality of cells or a large amount of DNA.

[3] The method described in [1] or [2], in which the weighted genotypic data are used to define a distance between the cells or between the cell groups and to determine a relationship of the distance between the cells or between the cell groups, thereby determining whether the cells or cell groups are derived from the same person, or unrelated persons, or a parent and a child, or persons in a blood relationship.

[4] The method described in any one of [1] to [3], in which the cells or cell groups are cells or cell groups isolated from peripheral blood of a pregnant woman.

[5] The method described in any one of [1] to [4], in which the weight distribution is corrected with reference to a genotype frequency in a population.

[6] The method described in any one of [1] to [4], in which the weight distribution is corrected with reference to an established genotype of a father and/or a mother.

[7] The method described in any one of [2] to [6], in which in a case where the cells or cell groups are derived from any one of a mother and a fetus, provided that there is data indicating presence of Y chromosome in observed genotypic data, the cells or cell groups are estimated to be derived from the fetus.

[8] The method described in [3], in which the distance is a likelihood or a posterior probability.

[9] The method described in any one of [1] to [7], further comprising:

a step of calculating a distance between cells, the cells constituting a cell group that has a possibility of being derived from N persons; and

a step of clustering the cells depending on a chance of being the same person and obtaining a final cluster number k, thereby determining that in a case where k = 1, the cell group is composed of cells derived from the same person, in a case where $k \neq 1$ and $k \leq N$, the cell group is composed of cells derived from k persons in N persons, and in a case where $k \neq 1$ and $k > N$, the cell group is composed of cells derived from k persons including a person other than N persons, where each of N and k is an integer of 1 or more.

According to the present invention, it is possible to provide a method for determining whether cells or cell groups that have a possibility of being derived from a plurality of persons are derived from the same person, or unrelated persons, or a parent and a child, or persons in a blood relationship, the method being capable of determining, with high accuracy, whether the cells or cell groups are derived from the same person, or unrelated persons, or a parent and a child, or persons in a blood relationship, even in a case where errors due to DNA amplification and sequencing are large and errors frequently occur in thus obtained polymorphism information of genes in genetic analysis for a small amount of deoxyribonucleic acid (DNA).

**BRIEF DESCRIPTION OF THE DRAWINGS**

Fig. 1 is a schematic diagram illustrating a method of acquiring weighted genotypic data from observed genotypic data.
Fig. 2 is a graph showing a relationship between a depth ratio (horizontal axis: depth ratio) of an observed base and a conditional probability (vertical axis: conditional probability) that the observed base is heterozygous as a true genotype.

Fig. 3 is a graph showing a relationship between a depth ratio (horizontal axis: depth ratio) of an observed base and a conditional probability (vertical axis: conditional probability) that the observed base is homozygous as a true genotype.

Fig. 4 is a dendrogram showing a hierarchical clustering performed in Example 1. A numerical value attached to a branch (lineage) is a distance between clusters (representing a chance of being the same person).

## DESCRIPTION OF THE PREFERRED EMBODIMENTS

First, features of the present invention which are characteristic compared to the related art will be described.

An invention described in WO2012/088456A relates to a method of using genetic information obtained from peripheral blood of an alleged father and a pregnant woman to evaluate whether or not the alleged father is a biological father of a fetus in the pregnant woman. Specifically, the invention described in WO2012/088456A relates to a method which includes acquiring genetic information such as a single nucleotide polymorphism (SNP) of the alleged father from the alleged father, acquiring genetic information such as an SNP of the pregnant woman and the fetus from peripheral blood of the pregnant woman, so as to (a) determine a probability that the alleged father is a biological father of the fetus, and (b) determine whether or not the alleged father is a biological father of the fetus based on the probability. However, this method is not intended to target single cells. Thus, it is difficult to differentiate individual cells from each other depending on their origin, in a situation where maternal cells and fetal cells may be mixed with each other.

In contrast, the present invention relates to a method for differentiating individual cells from each other depending on their origin by using genetic information such as a single nucleotide polymorphism (SNP), in a situation where cells of different origins may be mixed. More specifically, the present invention is different from the related art as described above in that genetic information such as an SNP is acquired from a cell group which is isolated from peripheral blood of a pregnant woman and in which maternal cells and fetal cells may be mixed with each other, weighting is performed on each genotype in consideration of the genotype and an appearance frequency with respect to gene polymorphism sites, and weighted genotypic data obtained by weighting genotypic data are used to determine whether cells or cell groups are derived from the same person, or unrelated persons, or a parent and a child, or persons in a blood relationship. As a result, in the present invention, it is possible to determine, with high accuracy, whether cells or cell groups are derived from the same person, or unrelated persons, or a parent and a child, or persons in a blood relationship, even with respect to a single cell for which errors due to DNA amplification and sequencing are large.

Hereinafter, the method of the present invention for determining whether cells or cell groups are derived from the same person, or unrelated persons, or a parent and a child, or persons in a blood relationship will be described in detail.

The method of the present invention for determining whether cells or cell groups are derived from the same person, or unrelated persons, or a parent and a child, or persons in a blood relationship includes a step of acquiring genotypic data for gene polymorphism sites of the cells or cell groups and weighting the data with a preset weight distribution, to acquire weighted genotypic data, and a step of using the weighted genotypic data to determine whether the cells or cell groups are derived from the same person, or unrelated persons, or a parent and a child, or persons in a blood relationship, in which the preset weight distribution is set by mapping of a measured apparent genotype and a true genotype with respect to the gene polymorphism sites.

<Description of each step>

Each step will be described below.

<<Step of acquiring genotypic data for gene polymorphism sites of cells or cell groups and weighting data with preset weight distribution, to acquire weighted genotypic data>>

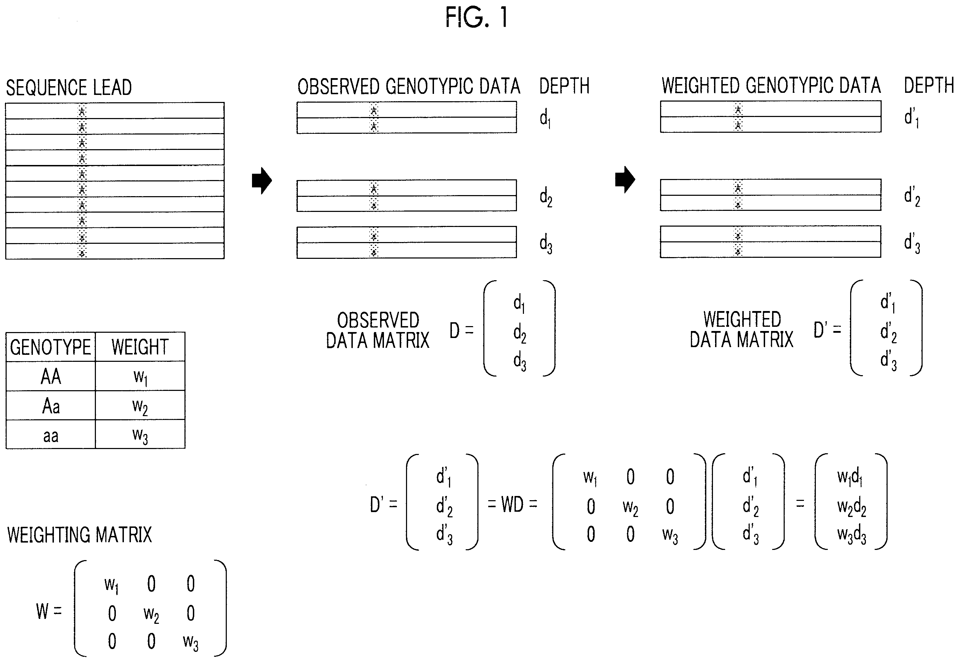

This step will be described with reference to Fig. 1 where appropriate.

In an example shown in Fig. 1, weights $w_1$, $w_2$, and $w_3$ are assigned to genotypes AA, Aa, and aa, respectively.

Observed genotypic data (depth ratio) are weighted with a preset weight distribution to acquire weighted genotypic data (depth ratio). In the example shown in Fig. 1, depths $d_1$, $d_2$, and $d_3$ of the observed genotypes are calculated from a sequence lead, are represented as an observed data matrix D, depths $d'_1$, $d'_2$, and $d'_3$ of the weighted genotypes are represented as a weighted data matrix D', and D' is calculated as a product WD of a weighting matrix W and the observed data matrix D.

<<Step of determining whether cells or cell groups are derived from same person, or unrelated persons, or parent and child, or persons in blood relationship>>

In the step of using a weighted genotypic data to determine whether cells or cell groups are derived from the same person, or unrelated persons, or a parent and a child, or persons in a blood relationship, examples of a method for determining whether the cells or cell groups are derived from the same person, or unrelated persons, or a parent and a child, or persons in a blood relationship include a method of using a weighted genotypic data and based on a distance between cells or between cell groups, a method of clustering entire cells, and a method of using an evaluation based on a magnitude of a likelihood ratio.

In the method based on the distance between cells or between cell groups, weighted genotypic data (depth ratio) are used to define a distance between the cells or between the cell groups, and to determine whether the cells or cell groups are derived from the same person, or unrelated persons, or a parent and a child, or persons in a blood relationship, based on the distance. The distance between the cells or between the cell groups is preferably a likelihood or a posterior probability.

The likelihood refers to a probability that observed data occurs under a certain hypothesis or model. In addition, in a case where a result appears depending on a certain precondition, one in which a numerical value expressing the highest chance of conversely assuming "what the precondition was" in view of an observed result is taken as a function with "what" as a variable is called a likelihood function.

The posterior probability is a type of conditional probability and is a type of subjective probability that expresses a known degree of a certain variable as a probability under a condition where a certain evidence (data or information) is taken into consideration.

In the method of clustering entire cells, a determination as to whether the cells or cell groups are derived from the same person, or unrelated persons, or a parent and a child, or persons in a blood relationship is performed by dividing a set of data into a plurality of subsets (clusters) based on a similarity between the data.

In the present invention, either a hierarchical clustering or a non-hierarchical clustering may be used for clustering.

In the hierarchical clustering, individual data are set as one cluster, a similarity or dissimilarity between clusters is calculated and the most similar clusters are merged, and the merging is repeated until all the clusters become one cluster. Examples of a method for obtaining the similarity or dissimilarity between clusters include a nearest neighbor method in which a distance between the nearest data is defined as a distance between two clusters, a furthest neighbor method in which a distance between the furthest data is defined as a distance between two clusters, and a centroid method in which the center of each of two clusters is obtained and a distance between the centers is defined as a distance between clusters. As a distance between the data, the above-described likelihood, posterior probability, or the like may be used.

In the non-hierarchical clustering, it is possible to perform a search using a function representing a state of division so that a value of the function is an optimal solution, which is also called a division optimization method. Examples of the non-hierarchical clustering method include a k-means method in which an average of clusters is used to classify data into k clusters which are given.

The hierarchical clustering method will be described in more detail. For example, in a case where there are n data of $D_1$, $D_2$, ⋯, $D_n$, a numerical value $d_{ij}$ (1, 2, ⋯, n) representing a degree of similarity between data $D_i$ and $D_j$ can be obtained. Here, $d_{ij}$ is symmetric ($d_{ij} = d_{ji}$). For an index indicating a degree of similarity, a smaller value may indicate a higher similarity such as a distance, and a larger value may indicate a higher similarity such as a correlation coefficient. Both may be collectively referred to as a similarity. Here, however, distinction is made by designating the former index as a dissimilarity, and the latter index as a similarity. Hereinafter, for simplicity, $d_{ij}$ represents a dissimilarity, and a smaller value indicates a higher similarity. As the dissimilarity, a distance is representative and preferable. The hierarchical cluster analysis method is an analysis method aiming at constructing a dendritic classification structure called a tree diagram or dendrogram by using a dissimilarity ($d_{ij}$) between such targets as a clue. A predetermined number of clusters of 1 to n can be obtained by cutting the tree diagram at appropriate sections. At this time, a cluster composed of a small number of constitutional units, which is obtained by cutting at a position close to the tip of a branch, is contained as it is in a cluster consisting of a large number of constitutional units, which is obtained by cutting at a root of a larger branch to which the branch is attached. That is, clusters that are obtained by cutting at various sections of a tree diagram have a hierarchical structure of minor classification - medium classification - ⋯ - major classification.

A process of agglomerative hierarchical cluster analysis generally includes the following steps.

Step 1: Start from n clusters each having one target as a constitutional unit.
Step 2: Refer to a dissimilarity matrix ($d_{ij}$) between clusters and fuse two clusters with the highest similarity together to form one cluster.
Step 3: In a case where the number of clusters is 1, end the process. Otherwise, proceed to the next step.

Step 4: Calculate a dissimilarity between the cluster newly created in step 2 and the other clusters so as to update the dissimilarity matrix ($d_{ij}$), and return to step 2.

In the method using an evaluation based on a magnitude of a likelihood ratio, for example, a weighted depth ratio is multiplied by a likelihood function for all gene polymorphism sites to be targeted so that a likelihood of each genotype of the gene polymorphism sites is calculated, the maximum likelihood with/without constraints is calculated, a likelihood ratio is obtained, and based on a magnitude of the likelihood ratio, it is possible to determine whether the cells or cell groups are derived from the same person, or unrelated persons, or a parent and a child, or persons in a blood relationship.

In the present invention, it is preferable to determine whether cells or cell groups are derived from the same person, or unrelated persons, or a parent and a child, or persons in a blood relationship, by defining a distance between cells or between cell groups using weighted genotypic data, and determining a genetic positional relationship between the cells or between cell groups.

The genetic positional relationship refers to a genetic link between cells, in which being closer to each other indicates a closer relationship. A more specific example thereof is a distance between cells, and the distance is more preferably a likelihood or a posterior probability.

<Cells or cell groups>

Cells that may be derived from a plurality of persons are not particularly limited, and it is preferable to perform a genotypic analysis for a single cell, for example, nucleated cells in peripheral blood of a pregnant woman, particularly nucleated red blood cells, can be mentioned because there is a high possibility that fetal cells and maternal cells are mixed. In a case of cells or cell groups isolated from peripheral blood of a pregnant woman, problems of allelic drop-out and allelic drop-in are necessarily involved in determination methods of the related art. However, according to the present invention, it is possible to make a more accurate determination. In addition, a cell group means a set of cells including one or more cells.

<Gene polymorphism site>

The gene polymorphism site is not particularly limited as long as it includes a locus where a gene polymorphism exists. There are no particular limitations on the gene polymorphism, and examples thereof include a single nucleotide polymorphism (SNP), a short tandem repeat (STR), and a copy number variation (CNV).

In the method of the present invention for determining whether cells or cell groups are derived from the same person, or unrelated persons, or a parent and a child, or persons in a blood relationship, the gene polymorphism site preferably includes one or both of a single nucleotide polymorphism site and a copy number variation site, and more preferably includes a single nucleotide polymorphism site. The single nucleotide polymorphism is widely used in a technical field associated with determining whether cells or cell groups are derived from the same person, or unrelated persons, or a parent and a child, or persons in a blood relationship. This is because it has advantages such as data accumulation and fidelity of an analysis method in implementing the present invention.

Further, in the method of the present invention for determining whether cells or cell groups are derived from the same person, or unrelated persons, or a parent and a child, or persons in a blood relationship, it is more preferable that the gene polymorphism site is a single nucleotide polymorphism site and the weight distribution is set in consideration of haplotypes of a plurality of single nucleotide polymorphism sites. This is because possible genotypes may be limited in a haplotype.

<Weight distribution>

The weight distribution is set by mapping an apparent genotype with a true genotype with respect to the gene polymorphism site. This setting is preferably made by specifying or estimating the mapping of the apparent genotype and the true genotype from results of experiments and/or simulations using a plurality of cells or a large amount of DNA.

Description will be made below by way of examples of cases.

<Case 1> Example where relationship between observed genotype and true genotype is specified using probability

Regarding a certain single nucleotide polymorphism (SNP) site X, there are two possibilities of genotypes $p_1q_1$ and $p_2q_2$. In addition, according to precise experiments, at the SNP site X, it is known that in a case where a true genotype is $p_1q_1$, a probability $P(B_1|A_1)$ that an apparent genotype is $p_1q_1$ is 0.90, and a probability $P(B_2|A_2)$ that the apparent genotype is $p_2q_2$ is 0.10; and in a case where the true genotype is $p_2q_2$, a probability $P(B_2|A_2)$ that the apparent genotype

is $p_2q_2$ is 0.70, and a probability $P(B_1|A_2)$ that the apparent genotype is $p_1q_1$ is 0.30. Here, $A_1$ is an event in which the true genotype is $p_1q_1$, $A_2$ is an event in which the true genotype is $p_2q_2$, $B_1$ is an event in which the apparent genotype is $p_1q_1$, and $B_2$ is an event in which the apparent genotype is $p_2q_2$. In addition, the prior probability $P(A_1) = P(A_2) = 0.50$.

Under this condition, the probability $P(A_1|B_1)$ that the true genotype is $p_1q_1$ in a case where the apparent genotype is $p_1q_1$, and probability $P(A_2|B_2)$ that the true genotype is $p_2q_2$ in a case where the apparent genotype is $p_2q_2$ are obtained.

According to Bayes' theorem, $P(A_i|B_j) = \{P(A_i)P(B_j|A_i)\}/\{\Sigma_k P(A_k)P(B_j|A_k)\}$. Thus, the following equation is obtained.

$$P(A_1|B_1) = \{P(A_1)P(B_1|A_1)\}/\{\Sigma_k P(A_k)P(B_1|A_k)\} = (0.50 \times 0.90)/(0.50 \times 0.90 + 0.50 \times 0.30) = 0.75$$

$$P(A_2|B_2) = \{P(A_2)P(B_2|A_2)\}/\{\Sigma_k P(A_k)P(B_2|A_k)\} = (0.5 \times 0.7)/(0.5 \times 0.1 + 0.5 \times 0.7) = 0.875$$

That is, the probability $P(A_1|B_1)$ that the true genotype is $p_1q_1$ in a case where the apparent genotype is $p_1q_1$ is 0.75, and probability $P(A_2|B_2)$ that the true genotype is $p_2q_2$ in a case where the apparent genotype is $p_2q_2$ is 0.875.

Therefore, in cases where the apparent genotype is $p_1q_1$ and where the apparent genotype is $p_2q_2$, probabilities that these match the true genotypes are 0.75 and 0.875, respectively.

$P(A_1|B_1)$ and $P(A_2|B_2)$ are used for weighting.

<Case 2> Example where relationship between true genotype and apparent genotype is specified using variation

As a genotype of a certain single nucleotide polymorphism (SNP) locus Y, there are four possibilities of $r_1s_1$, $r_1s_2$, $r_2s_1$, and $r_2s_2$. In addition, data obtained by normalizing a depth obtained for each genotype which may be a genotype of the SNP locus Y with a total depth is set as $(d_1, d_2, d_3, d_4)$. Here, $0 \leq d_1 \leq 1$, $0 \leq d_2 \leq 1$, $0 \leq d_3 \leq 1$, $0 \leq d_4 \leq 1$, and $d_1 + d_2 + d_3 + d_4 = 1$.

Ideally, in a case where the true genotype is $r_1s_1$, $(d_1, d_2, d_3, d_4) = (1, 0, 0, 0)$. However, particularly in a case of single cell analysis, due to effects of an allelic drop-out (ADO) and an allelic drop-in (ADI), variations such as $(d_1, d_2, d_3, d_4) = (0.8, 0.05, 0.05, 0.1)$ occur.

Distributions of the true genotype and $(d_1, d_2, d_3, d_4)$ are estimated as follows.

i) Estimation method of distribution - Estimation method by experiments

A distribution of $(d_1, d_2, d_3, d_4)$ is estimated experimentally.

First, DNA amplification and sequencing are performed using a large amount of deoxyribonucleic acid (DNA), and a true genotype is established. Next, DNA amplification and sequencing experiments are performed a plurality of times using a single cell to estimate a distribution of $(d_1, d_2, d_3, d_4)$ and the true genotype.

It may be assumed that there is no difference between cells, between individuals, or between regions of PCR reaction, or the difference may be taken into consideration.

ii) Estimation method of distribution - Estimation method by simulations

The distribution of $(d_1, d_2, d_3, d_4)$ is estimated by simulations.

First, models for DNA amplification, sequencing, or the like are constructed. Next, genotypes are assumed and $(d_1, d_2, d_3, d_4)$, which is to be obtained at that time, is acquired in plural by using Monte Carlo simulation to estimate the distribution.

In a case of constructing the models, it may be assumed that there is no difference between cells, between individuals, or between regions of PCR reaction, or the difference may be taken into consideration.

iii) Estimation method of distribution - Estimation method by experiments and simulations

The distribution of $(d_1, d_2, d_3, d_4)$ is estimated by experiments and simulations.

First, parameters are estimated according to the above-described method of estimating distribution by experiments, and models for DNA amplification, sequencing, or the like are constructed.

Next, a genotype is assumed and $(d_1, d_2, d_3, d_4)$, which is to be obtained at that time, is acquired in plural by using Monte Carlo simulation to estimate the distribution.

The distribution estimated in this manner is used for weighting.

<Case 3> Example where relationship between observed genotype and true genotype is specified using conditional probability of true genotype when depth is obtained

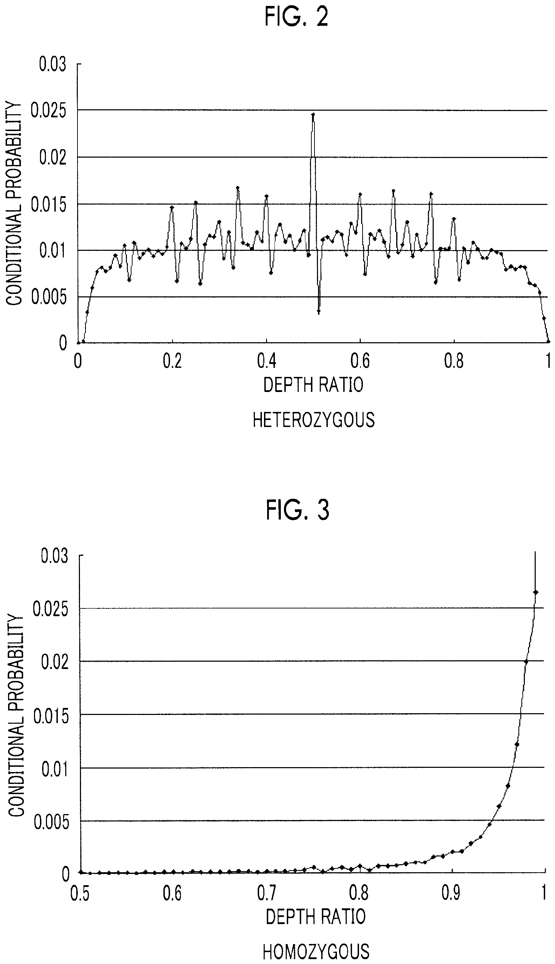

Description will be made with reference to Figs. 2 and 3.

Fig. 2 is a graph showing a relationship between a depth ratio of an observed base and a conditional probability that the observed base is heterozygous as a true genotype.

For example, in a case where 0.5 is observed as a depth ratio of a base C at a certain single nucleotide polymorphism (SNP) position Z, a conditional probability that C is heterozygous as a true genotype is about 0.025 as shown in Fig. 2.

Further, Fig. 3 is a graph showing a relationship between a depth ratio of an observed base and a conditional probability that the observed base is homozygous as a true genotype.

For example, in a case where 0.98 is observed as a depth ratio of a base C at a certain SNP position Z, a conditional probability that C is homozygous as a true genotype is about 0.02 as shown in Fig. 3.

<Case 4> Example of experimental mapping

Cell lysis and genome extraction are performed on a large number of cultured cells (about $10^6$ cells) using a commercially available extraction kit for genomic deoxyribonucleic acid (DNA).

For about 10 ng of the obtained genome, a region containing a desired single nucleotide polymorphism (SNP) is amplified by polymerase chain reaction (PCR) and sequenced using a next-generation sequencer. Furthermore, an output of the sequencer is aligned/mapped with a reference genome using an alignment tool/mapping tool such as BWA (Burrows-Wheeler Aligner) (Bioinformatics, 2009, 25(14): 1754 to 1760; Bioinformatics, 2010, 26(5): 589 to 595; http://bio-bwa.sourceforge.net/) to obtain depth information of a desired area. Furthermore, each locus is counted using SAMtools (SAM: Sequence Alignment/Map) (Bioinformatics, 2009, 25(16): 2078 to 2079; http://github.com/samtools/samtools) to acquire depth information, and an SNP call is performed using BCFtools (BCF: Binary Call Format) (http://github.com/samtools/bcftools) to determine a genotype at each SNP with respect to a large number of cells.

A similar operation is carried out for about 100 cells in isolated single cells to obtain a distribution of depth.

Mapping of a depth in a single cell and a true genotype is performed using a distribution of a true genotype and a depth at each of the obtained SNPs.

<Case 5> Example of mapping by simulations

An example of an amplification model is illustrated. For a certain single nucleotide polymorphism (SNP) position, a ratio of (A, C, G, T) after an $r^{th}$ polymerase chain reaction (PCR) is defined as (A(r), C(r), G(r), T(r)). In this case, an initial value (A(0), C(0), G(0), T(0)) is, for example, (2, 0, 0, 0) in a case of being A homozygous, and (1, 1, 0, 0) in a case of being AC heterozygous. Here, r is an integer of 1 or more.

In an $r+1^{th}$ PCR, denaturation, annealing, and extension are performed on a fragment of each deoxyribonucleic acid (DNA) after the $r^{th}$ PCR. In this case, in consideration of a probability p ($0 \le p \le 1$) that a primer anneals to a DNA single strand and a probability q ($0 \le p \le 1$) that a correct base synthesis is performed, (A(r + 1), C(r + 1), G(r + 1), T(r + 1)), which is a ratio of (A, C, G, T) after an $r+1^{th}$ PCR, is as follows.

$$A(r + 1) = A(r) * p * q + C(r) * p * (1 - q)/3 + G(r) * p * (1 - q)/3 + T(r) * p * (1 - q)/3$$

$$C(r + 1) = C(r) * p * q + A(r) * p * (1 - q)/3 + G(r) * p * (1 - q)/3 + T(r) * p * (1 - q)/3$$

$$G(r + 1) = G(r) * p * q + A(r) * p * (1 - q)/3 + C(r) * p * (1 - q)/3 + T(r) * p * (1 - q)/3$$

$$T(r + 1) = T(r) * p * q + A(r) * p * (1 - q)/3 + C(r) * p * (1 - q)/3 + G(r) * p * (1 - q)/3$$

In parameters p and q of the model, there are many locations where an initial value is homozygous. Thus, a correspondence between a true genotype and a depth is estimated by experiments using a large amount of cells with respect to the locations where the initial value is homozygous, and the most fitting value is estimated by Kolmogorov-Smirnov test (KS test). Mapping of a true base-type and a depth in a case where an initial value is heterozygous is estimated using the obtained p and q by Monte Carlo simulation. The graphs shown in Figs. 2 and 3 correspond to result examples, respectively, for a case where the initial value is heterozygous and a case where the initial value is homozygous.

<Correction of weight distribution or weighted genotypic data>

<<Correction with reference to genotype frequency in population>>

In the method of the present invention for determining whether cells or cell groups are derived from the same person, or unrelated persons, or a parent and a child, or persons in a blood relationship, it is preferable to correct a weight distribution with reference to a genotype frequency in a population.
This is because a bias of a genotype frequency for each population is incorporated into weighting. Variations between races and between ethnicities may be particularly large, depending on a locus and a genotype.

<<Correction with reference to established genotype of father and/or mother>>

In the method of the present invention for determining whether cells or cell groups are derived from the same person, or unrelated persons, or a parent and a child, or persons in a blood relationship, it is preferable to correct a weight distribution with reference to an established genotype of a father and/or a mother.
This is because a genotype of a child may be limited by a genotype of a father and/or a mother. In addition, in a case where an apparent genotypic data of the child is greatly different from a genotypic data predicted from a genotype of the father and/or the mother, contamination is suspected, and thus a determination can be made not to perform a subsequent analysis.

<<Correction using plurality of single cell data>>

In the method of the present invention for determining whether cells or cell groups are derived from the same person, or unrelated persons, or a parent and a child, or persons in a blood relationship, it is preferable to correct a weight distribution using a plurality of single cell data. This is because it is expected that a more appropriate weighting can be performed.

<Presence of Y chromosome>

Further, in the method of the present invention for determining whether cells or cell groups are derived from the same person, or unrelated persons, or a parent and a child, or persons in a blood relationship, it is preferable that in a case where cells or cell groups that have a possibility of being derived from a plurality of persons are derived from any one of a mother and a fetus, provided that there is data indicating presence of Y chromosome in observed genotypic data, the cells or cell groups are estimated to be derived from the fetus.
Maternal cells only have X chromosome as a sex chromosome. Thus, the presence of Y chromosome is a strong evidence that cells from which the genotypic data have been obtained are derived from the fetus.

<Clustering>

Further, the method of the present invention for determining whether cells or cell groups are derived from the same person, or unrelated persons, or a parent and a child, or persons in a blood relationship, preferably further includes a step of calculating a distance between cells, the cells constituting a cell group that have a possibility of being derived from N persons, and a step of clustering the cells depending on a chance of being the same person and obtaining a final cluster number k, thereby determining that in a case where k = 1, the cell group is composed of cells derived from the same person, in a case where k ≠ 1 and k ≤ N, the cell group is composed of cells derived from k persons in N persons, and in a case where k ≠ 1 and k > N, the cell group is composed of cells derived from k persons including a person other than N persons. Here, each of N and k is an integer of 1 or more.
As a clustering method, any of the above-mentioned methods can be used.

Examples

[Example 1]

(Subject)

Three families which are Families C, D, and E were targeted. As subjects, two persons who are a son (symbol: Cson) and a mother (symbol: Cmom) were selected from Family C; three persons who are an elder sister (symbol: Dsis1), a younger sister (symbol: Dsis2), and a mother (symbol: Dmom) were selected from Family D; and three persons who are a son (symbol: Eson), a mother (symbol: Emom), and a father (symbol: Edad) were selected from Family E.

(Experimental method)

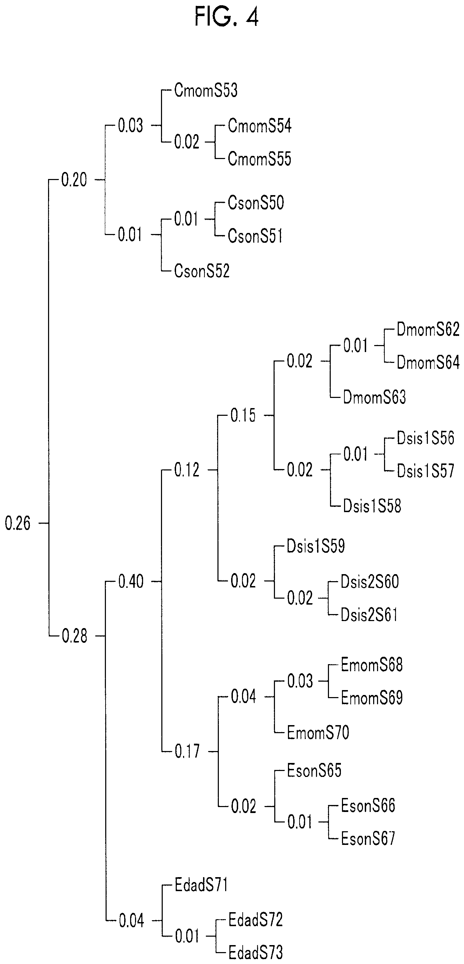

Analysis was performed for a single nucleotide polymorphism (SNP) on chromosome 13, chromosome 18, chromosome 21, and X chromosome. A hierarchical clustering was performed using obtained weighted genotypic data. Dendrogram thus obtained is shown in Fig. 4. A numerical value attached to a branch (lineage) indicates a distance between clusters. A smaller distance indicates a greater chance of being the same person.

(Result and discussion)

The distance becomes larger in the order of the same person < a parent and a child < persons in a blood relationship (blood relationship other than a parent and a child) < unrelated persons (unrelated persons having no blood relationship).
Because X chromosome is observed, the father in Family E (symbol: Edad) is away from the son (symbol: Eson) and the mother (symbol: Emom) in Family E.
The present invention is particularly useful for mother-child identification, parent-child identification or blood relationship identification in prenatal diagnosis.

**Claims**

1. A method for determining whether cells or cell groups that have a possibility of being derived from a plurality of persons are derived from the same person, or unrelated persons, or a parent and a child, or persons in a blood relationship, the method comprising:

   a step of acquiring genotypic data for gene polymorphism sites of the cells or cell groups and weighting the data with a preset weight distribution, to acquire weighted genotypic data; and
   a step of using the weighted genotypic data to determine whether the cells or cell groups are derived from the same person, or unrelated persons, or a parent and a child, or persons in a blood relationship,
   wherein the preset weight distribution is set by mapping of a measured apparent genotype and a true genotype with respect to the gene polymorphism sites.

2. The method according to claim 1,
   wherein the mapping of the apparent genotype and the true genotype is estimated by experiments and/or simulations using a plurality of cells or a large amount of DNA.

3. The method according to claim 1 or 2,
   wherein the weighted genotypic data are used to define a distance between the cells or between the cell groups and to determine a genetic positional relationship between the cells or between the cell groups, thereby determining whether the cells or cell groups are derived from the same person, or unrelated persons, or a parent and a child, or persons in a blood relationship.

4. The method according to any one of claims 1 to 3,
   wherein the cells or cell groups are cells or cell groups isolated from peripheral blood of a pregnant woman.

5. The method according to any one of claims 1 to 4,
   wherein the weight distribution is corrected with reference to a genotype frequency in a population.

**6.** The method according to any one of claims 1 to 4,
wherein the weight distribution is corrected with reference to an established genotype of a father and/or a mother.

**7.** The method according to any one of claims 1 to 6,
wherein in a case where the cells or cell groups are derived from any one of a mother and a fetus, provided that there is data indicating presence of Y chromosome in observed genotypic data, the cells or cell groups are estimated to be derived from the fetus.

**8.** The method according to claim 3,
wherein the distance is a likelihood or a posterior probability.

**9.** The method according to any one of claims 1 to 7, further comprising:

a step of calculating a distance between cells, the cells constituting a cell group that has a possibility of being derived from N persons; and
a step of clustering the cells depending on a chance of being the same person and obtaining a final cluster number k, thereby determining that in a case where k = 1, the cell group is composed of cells derived from the same person, in a case where k ≠ 1 and k ≤ N, the cell group is composed of cells derived from k persons in N persons, and in a case where k ≠ 1 and k > N, the cell group is composed of cells derived from k persons including a person other than N persons, where each of N and k is an integer of 1 or more.

# FIG. 1

SEQUENCE LEAD

| GENOTYPE | WEIGHT |
|----------|--------|
| AA | $w_1$ |
| Aa | $w_2$ |
| aa | $w_3$ |

WEIGHTING MATRIX

$$W = \begin{pmatrix} w_1 & 0 & 0 \\ 0 & w_2 & 0 \\ 0 & 0 & w_3 \end{pmatrix}$$

OBSERVED GENOTYPIC DATA   DEPTH

$d_1$

$d_2$

$d_3$

OBSERVED DATA MATRIX   $D = \begin{pmatrix} d_1 \\ d_2 \\ d_3 \end{pmatrix}$

WEIGHTED GENOTYPIC DATA   DEPTH

$d'_1$

$d'_2$

$d'_3$

WEIGHTED DATA MATRIX   $D' = \begin{pmatrix} d'_1 \\ d'_2 \\ d'_3 \end{pmatrix}$

$$D' = \begin{pmatrix} d'_1 \\ d'_2 \\ d'_3 \end{pmatrix} = WD = \begin{pmatrix} w_1 & 0 & 0 \\ 0 & w_2 & 0 \\ 0 & 0 & w_3 \end{pmatrix} \begin{pmatrix} d'_1 \\ d'_2 \\ d'_3 \end{pmatrix} = \begin{pmatrix} w_1 d_1 \\ w_2 d_2 \\ w_3 d_3 \end{pmatrix}$$

EP 3 375 886 A1

## FIG. 2

HETEROZYGOUS

## FIG. 3

HOMOZYGOUS

# FIG. 4

## INTERNATIONAL SEARCH REPORT

| International application No. |
| --- |
| PCT/JP2016/081476 |

### A. CLASSIFICATION OF SUBJECT MATTER
*C12Q1/68*(2006.01)i, *C12N15/09*(2006.01)i

According to International Patent Classification (IPC) or to both national classification and IPC

### B. FIELDS SEARCHED

Minimum documentation searched (classification system followed by classification symbols)
C12Q1/68, C12N15/09

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/MEDLINE/BIOSIS/WPIDS(STN)

### C. DOCUMENTS CONSIDERED TO BE RELEVANT

| Category* | Citation of document, with indication, where appropriate, of the relevant passages | Relevant to claim No. |
| --- | --- | --- |
| A | JP 2014-502845 A (Natera, Inc.), 06 February 2014 (06.02.2014), & JP 2014-502845 A & US 2011/0288780 A1 & WO 2012/088456 A2 & EP 2656263 A2 | 1-9 |
| A | JP 2009-517050 A (Gene Security Network LLC), 30 April 2009 (30.04.2009), & US 2007/0027636 A1 & WO 2007/062164 A2 & EP 1960929 A | 1-9 |
| A | JP 2012-502631 A (Sequenom, Inc.), 02 February 2012 (02.02.2012), & US 2010/0105049 A1 & WO 2010/033639 A2 & EP 2329021 A2 | 1-9 |

☐ Further documents are listed in the continuation of Box C.     ☐ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| 13 January 2017 (13.01.17) | 24 January 2017 (24.01.17) |

| Name and mailing address of the ISA/ <br> Japan Patent Office <br> 3-4-3,Kasumigaseki,Chiyoda-ku, <br> Tokyo 100-8915,Japan | Authorized officer <br><br> Telephone No. |
| --- | --- |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012088456 A

**Non-patent literature cited in the description**

- *Bioinformatics,* 2009, vol. 25 (14), 1754-1760
- *Bioinformatics,* 2010, vol. 26 (5), 589-595
- *Bioinformatics,* 2009, vol. 25 (16), 2078-2079